# EUROPEAN PATENT APPLICATION

(11) **EP 3 305 347 A1**
(43) Date of publication of application: **11.04.2018**
(21) Application number: 16799316.1
(22) Date of filing: 25.05.2016
(51) Int. Cl.: A61M 5/178, A61M 5/315, A61M 5/32, A61M 5/142

(54) **FLUID INFUSION APPARATUS USED FOR ADMINISTERING MEDICAMENT TO PATIENT**

(30) Priority: 25.05.2015 CN 201510270355
(71) Applicant: Medtronic Inc., Minneapolis, MN 55432 (US)
(72) Inventor: LI, Frank, Shanghai 201203 (CN); XUE, Yueqiang, Shanghai (CN); FENG, Wind, Shanghai 201203 (CN)
(74) Representative: Ruschke, Hans Edvard
(86) International application number: PCT/CN2016/083286
(87) International publication number: WO 2016/188428

(57) **Abstract**

A fluid infusion apparatus (100) used for administering a medication to a patient comprises: a reservoir (101) used for storing infusion fluid; a plunger (102) located within the reservoir (101) and limiting the infusion fluid together with the reservoir (101), wherein the plunger (102) is configured to be movable along the reservoir (101); an injection button (103) operatively and synchronously moving with the plunger (102) and configured to be operable; and a displacement limiting mechanism arranged parallel to the reservoir (101), and configured to limit the plunger (102) to move for a predetermined distance within the reservoir (101) when the injection button (103) is operated, thus dispensing a predetermined amount of the fluid stored in the reservoir (101). Implementing the above apparatus reduces the overall size of the apparatus and simplifies the setting process of the injection amount, thus facilitating the administration for users.

## Description

This application claims priority to Chinese Patent Application No. 201510270355.7, filed with the State Intellectual Property Office of P.R.C on May 25, 2015 and entitled "FLUID INFUSION APPARATUS USED FOR ADMINISTERING MEDICAMENT TO PATIENT", which is incorporated herein by reference in its entirety.

### TECHNICAL FIELD

The present invention relates to the field of fluid transportation, and in particular, to a fluid infusion apparatus capable of implementing a bolus injection and is used for administering a medicament to a patient.

### BACKGROUND

Diabetes is a metabolic disease featured with a high blood sugar level. The high blood sugar level is generally caused by insulin secretion deficiency or damaged biological effects thereof, or caused due to a combination of the two factors. The high blood sugar level existing in a diabetes patient for a long time will cause chronic damages and dysfunctions of various body organs (such as the eyes, the kidneys, the heart, the blood vessels, and the nervous system).

According to clinical diagnosis, the diabetes may be classified into type 1 diabetes and type 2 diabetes. The type 1 diabetes is also referred to as insulin-dependent diabetes mellitus and is a congenital familial hereditary disease. Symptoms of the patient generally appear during childhood or adolescence thereof. The type 1 diabetes belongs to an autoimmune disease, where an immune system of a body attacks β cells that produce insulin in the body, finally causing that the body unable to produce insulin. Such patients need to be injected with exogenous insulin to control the blood sugar level of the body. A patient of the type 1 diabetes generally needs to wear an electronic insulin pump, e.g., a Medtronic Minimed series insulin pump, for treatment all day long. The type 2 diabetes is also referred to as non-insulin dependent diabetes mellitus; and patients thereof are generally adults, especially obese people. The disease may cause emaciation; and the possible pathogenesis may include: insulin resistance, such that the body cannot effectively use the insulin; and reduced insulin secretion, such that the insulin cannot meet the requirement of the body. A patient at the early stage of the type 2 diabetes can control or even cure the diabetes by improving the life style (such as having a healthy diet, exercising moderately, losing weight appropriately, quitting smoking, and avoiding secondhand smoke). Most of the patients of the type 2 diabetes can control the blood sugar level of the body with the help of oral hypoglycemic drugs or control the blood sugar level by injecting insulin periodically. At present, most of the patients inject insulin by using insulin pen syringes. The insulin pen has a pen-like structure and includes three parts: a pen body, a pen holder, and a pen cap. Before insulin is injected, it is necessary to mount a matched insulin refill into the pen holder. Then, an adjustment button is rotated, and the injection amount is controlled by moving a screw. Then, an injection button is pressed, and the refill is driven by the screw to finish injection. In other words, using the existing insulin pen method requires a manual adjustment of the screw movment amount each time to control the infusion amount; and the setting process is a complicated one. Moreover, arranging the screw in the insulin pen increases the overall length of the apparatus, thus contradicting to the portable design use of the apparatus.

### SUMMARY

To solve the above technical problem, the present invention provides a fluid infusion apparatus used for administering a medicament to a patient, which can simply and quickly set the infusion amount, and can reduce the overall size of the apparatus.

According to a first aspect of embodiments of the present invention, a fluid infusion apparatus used for administering a medicament to a patient is provided; and the apparatus may comprise: a reservoir, used for storing infusion fluid; a plunger, located in the reservoir and limiting the infusion fluid together with the reservoir, wherein the plunger is configured to be movable along the reservoir; an injection button, operatively and synchronously moving with the plunger and configured to be operable; and a displacement limiting mechanism, arranged parallel to the reservoir, and configured to limit the plunger to move for a predetermined distance within the reservoir when the injection button is operated, thus dispensing a predetermined amount of the fluid stored in the reservoir.

In some embodiments, the displacement limiting mechanism may comprise: a base provided with a plurality of guide chutes having equal lengths; and a slider operatively connected to the injection button, and configured to be pushed to slide along a first guide chute in the plurality of guide chutes when the injection button is pressed, and to be disengaged from the first guide chute and slide to an adjacent second guide chute in the plurality of guide chutes when the injection button is released.

In some embodiments, the displacement limiting mechanism may further comprise: an energy storage component, operatively connected to the slider, and configured to store mechanical energy when the slider slides along the first guide chute and to release the mechanical energy such that the slider returns to an unpushed state when the slider slides to the second guide chute.

In some embodiments, the slider may comprise: a driving button operatively connected to the injection button and provided with a plurality of first protruding parts uniformly distributed along a circumference and embeddable into the guide chutes, wherein the first protruding part is provided with a driving slope; and a rotation sliding block, operatively coupled to the driving button and the energy storage component and provided with a plurality of second protruding parts uniformly distributed along the circumference and embeddable into the guide chutes, wherein the second protruding part is provided with a driven slope corresponding to the driving slope.

In some embodiments, the displacement limiting mechanism may comprise: a matching part, provided with a first opening and a second opening spaced at a fixed distance; and an elastic buckle, synchronously moving with the injection button and the plunger, and configured to pop out from the first opening when the injection button is pressed and move for the fixed distance to be assembled to the second opening.

In some embodiments, the matching part may further comprise: a reset button, matching with the second opening and configured to be pressed such that the elastic buckle pops out from the second opening.

In some embodiments, the displacement limiting mechanism may further comprise: an energy storage component, operatively connected to the elastic buckle, and configured to store mechanical energy when the elastic buckle moves from the first opening to the second opening, and to release the mechanical energy when the elastic buckle pops out from the second opening such that the elastic buckle moves for the fixed distance to be assembled to the first opening.

According to a second aspect of the embodiments of the present invention, a fluid infusion apparatus used for administering a medicament to a patient is provided; and the fluid infusion apparatus may comprise: a reservoir, used for storing to-be-infused fluid; an injection button, provided with a plunger movable along the reservoir; an injector, used for receiving the reservoir and being synchronously movable with the reservoir; a sealing element, arranged outside of the injector; and a first hollow piercing component, a base end of the first hollow piercing component being in communication with the reservoir, a front end of the first hollow piercing component being sealed within the sealing element, and the first hollow piercing component being synchronously movable with the reservoir, the injection button, and the injector, wherein when the injection button is pressed, the reservoir, the injector, and the first hollow piercing component are driven to move synchronously along a first direction such that the first hollow piercing component penetrates through the sealing element to dispense the fluid stored in the reservoir.

In some embodiments, the fluid infusion apparatus may further comprise: an energy storage component, operatively connected to the injector, and configured to store mechanical energy when the injection button is pressed and to release the mechanical energy when the injection button is released such that the first hollow piercing component is sealed within the sealing element.

In some embodiments, the fluid infusion apparatus may further comprise: a second hollow piercing component in operative communication with the reservoir, and being capable of inputting a fluid to the reservoir when it is in communication with the reservoir.

In some embodiments, the second hollow piercing component is further capable of retracting within the plunger when the injection button is pressed.

According to a third aspect of the embodiments of the present invention, a fluid infusion apparatus used for administering a medicament to a patient is provided; and the apparatus may comprise: a reservoir, used for storing infusion fluid; an output valve, operatively connected to an outlet of the reservoir; a flexible film covering the reservoir; and an injection button, located on the flexible film and provided with a filling element that fills the reservoir, and configured to be pressed such that the filling element, together with the flexible film, fills the reservoir, so that the fluid pressure within the reservoir forces open the output valve, enabling the fluid in the reservoir to be dispensed.

In some embodiments, the fluid infusion apparatus may further comprise: an input valve, operatively connected to an inlet of the reservoir; and a reset button, operatively connected to the injection button and configured to be pressed such that the filling element, together with the flexible film, is lifted from the reservoir, forcing the output valve to be closed and the input valve to be opened, thus inputting a fluid to the reservoir.

In some embodiments, the output valve may comprise a ball valve.

In some embodiments, the input valve may comprise a ball valve.

The fluid infusion apparatus provided in the embodiments of the present invention has the advantage that it reduces the overall size of the infusion apparatus such that the apparatus is as compact as possible to facilitate wearing for a user in various scenarios. Additionally, the fluid infusion apparatus helps the user to set the infusion amount in a convenient and fast manner.

### BRIEF DESCRIPTION OF THE DRAWINGS

FIG. 1 is a schematic sectional diagram of a fluid infusion apparatus used for administering a medicament to a patient according to a first embodiment of the present invention;
FIG. 2 is a schematic three-dimensional structural diagram of the fluid infusion apparatus used for administering a medicament to a patient according to the first embodiment of the present invention;
FIG. 3 is a schematic enlarged local sectional diagram according to the first embodiment of the present invention;
FIG. 4 is a schematic diagram of a free state of the fluid infusion apparatus used for administering a medicament to a patient according to the first embodiment of the present invention;
FIG. 5 is a schematic diagram of a working state of the fluid infusion apparatus used for administering a medicament to a patient according to the first embodiment of the present invention;
FIG. 6a is a schematic structural diagram of a fluid infusion apparatus used for administering a medicament to a patient according to a second embodiment of the present invention;
FIG. 6b is a schematic enlarged diagram of components according to the second embodiment of the present invention;
FIG. 7 is a schematic sectional diagram of a fluid infusion apparatus used for administering a medicament to a patient according to a third embodiment of the present invention;
FIG. 8a to FIG. 8e are schematic diagrams of operating states of the fluid infusion apparatus used for administering a medicament to a patient according to the third embodiment of the present invention;
FIG. 9 is a schematic diagram of a fluid infusion apparatus used for administering a medicament to a patient according to a fourth embodiment of the present invention; and
FIG. 10a and FIG. 10b are schematic diagrams of operating states of the fluid infusion apparatus used for administering a medicament to a patient according to the fourth embodiment of the present invention.

### DETAILED EMBODIMENTS

In order to make objectives, technical solutions, and advantages of the present invention more apparent and obvious, the present invention will be described in further detail with reference to the accompanying drawings. It should be noted that the present invention is not limited to component constructions and/or deployments shown in the accompanying drawings; and embodiments of the present invention may be combined in various manners without departing from the essence of the present invention.

The embodiments of the present invention provide a fluid infusion apparatus used for administering a medicament to a patient; and the fluid infusion apparatus may include a reservoir, a plunger, an injection button, and a displacement limiting mechanism. The reservoir may have a shape capable of storing infusion fluid, e.g., a receiving structure having an upper surface, a lower surface, and an outer surface. In some embodiments, the reservoir may be cylindrical. The infusion fluid that can be stored in the reservoir may be various types of liquid, gas, and the like used for administering a medicament to a patient. In some embodiments, the fluid may be a therapeutic liquid medicine such as insulin. The plunger is located in the reservoir and limits the infusion fluid together with the reservoir, and is configured to movable along the reservoir, thus pushing the fluid from the reservoir or sucking the fluid into the reservoir. In the case that the reservoir is cylindrical, the plunger is movable along an axial direction of the reservoir. The injection button moves operatively and synchronously with the plunger, and is operable; for example, the injection button can be pressed or released by a user. The displacement limiting mechanism is arranged parallel to the reservoir, and can be configured to limit the plunger to move for a predetermined distance within the reservoir when the injection button is operated (e.g., when the injection button is operated by pressing or releasing), thus dispensing a predetermined amount of the fluid corresponding to the predetermined distance stored in the reservoir. For example, the displacement limiting mechanism may be arranged outside or inside the outer surface of the reservoir; and a geometric virtual center line thereof is parallel to a geometric virtual center line of the reservoir. In the case that the reservoir is cylindrical, the plunger in the reservoir moves along an axis of the reservoir, and the displacement limiting mechanism is preferably parallel to the axis of the reservoir. Compared with the existing solution of directly adjusting the infusion amount of a reservoir by using a screw (in this solution, the screw being used as a displacement mechanism is in the same line with the reservoir, greatly increasing the size of the overall apparatus), arranging the displacement limiting mechanism parallel to the reservoir can reduce the overall size of the fluid infusion apparatus, such that the apparatus is more compact and facilitates wearing and use for a user in various scenarios (e.g., outdoor scenarios). Moreover, a distance of each move of the plunger is limited by the limiting mechanism to determine the infusion amount; and therefore, it requires no needs for manually adjusting a screw, thus simplifying the process for adjusting the infusion amount.

### [First Embodiment]

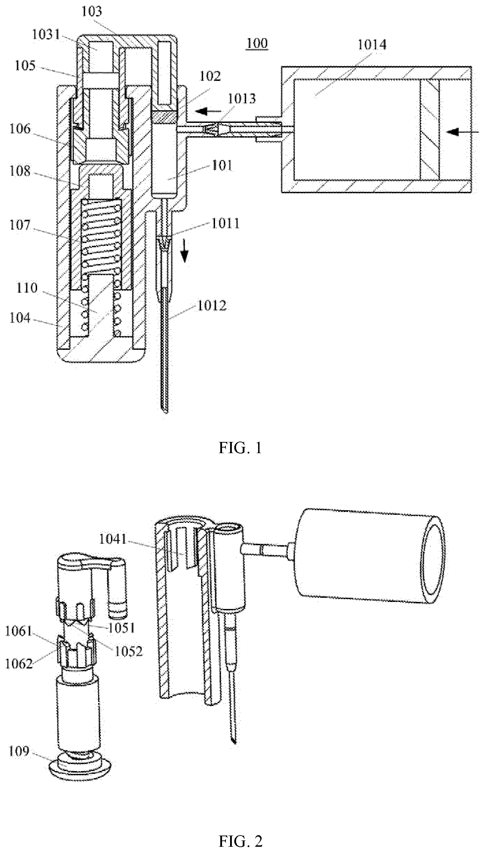

FIG. 1 is a schematic sectional diagram of a fluid infusion apparatus used for administering a medicament to a patient according to a first embodiment of the present invention; FIG. 2 is a schematic three-dimensional structural diagram of the fluid infusion apparatus used for administering a medicament to a patient according to the first embodiment of the present invention;

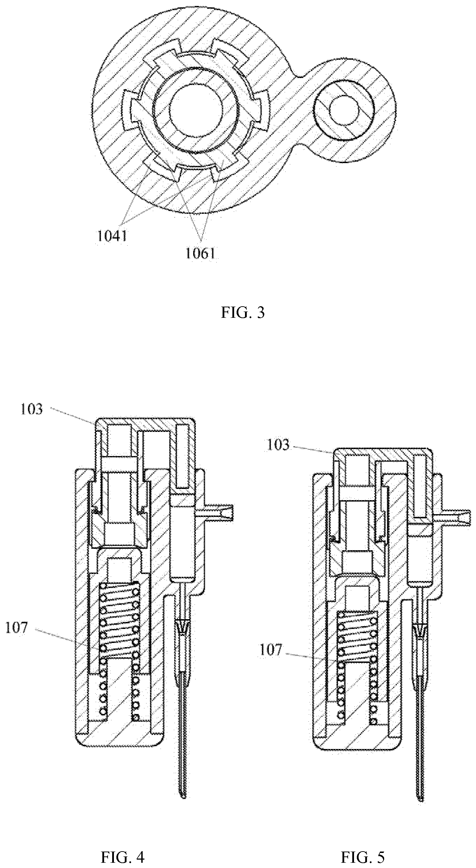

FIG. 3 is a schematic enlarged local sectional diagram according to the first embodiment of the present invention; FIG. 4 is a schematic diagram of a free state of the fluid infusion apparatus used for administering a medicament to a patient according to the first embodiment of the present invention; and FIG. 5 is a schematic diagram of a working state of the fluid infusion apparatus used for administering a medicament to a patient according to the first embodiment of the present invention.

As shown from FIG. 1 to FIG. 3, a fluid infusion apparatus 100 may include: a reservoir 101, a plunger 102, an injection button 103, a base 104, a driving button 105, a rotation sliding block 106, and a spring 107. The reservoir 101 may be a bolus reservoir storing a fluid amount used for one injection. Moreover, the reservoir (e.g., a cylindrical reservoir) 101 may be in communication with a hollow piercing component 1012 (such as a hollow steel needle) through a check valve 1011, such that when the plunger 102 moves downwards along an axial direction of the reservoir (viewing from the direction facing FIG. 1), the check valve 1011 is opened (e.g., along a direction pointed by an arrow at the right of the check valve 1011 in FIG. 1) to dispense the fluid in the reservoir 101 through the piercing component 1012, i.e., the fluid being transported to a patient. The reservoir 101 may further be in communication with a fluid supply room 1014 through a check valve 1013, and a push force (for example, a push force along a direction pointed by an arrow at the right of the fluid supply room 1014 in FIG. 1) may be applied to the fluid supply room 1014, to force the check valve 1013 to be opened along an arrow direction at the top, thus supplying the fluid to the reservoir 101.

As shown in FIG. 1 and FIG. 2, the injection button 103, the base 104, the driving button 105, the rotation sliding block 106, and the spring 107 may be arranged parallel to the reservoir 101. The base 104, the driving button 105, the rotation sliding block 106, and the spring 107 may together construct a displacement limiting mechanism, and a geometric center line of the displacement limiting mechanism may be parallel to a moving direction of the plunger 102 in the reservoir 101. The displacement limiting mechanism may cooperate with the injection button 103 to limit the plunger 102 to move for a predetermined distance within the reservoir 101. The injection button 103 is a linkage button that can act on the plunger 102 and the driving button 105 simultaneously and has a cylindrical structure 1031, and is arranged such that an axis direction of the cylindrical structure 1031 (i.e., the geometric center line direction of the displacement limiting mechanism) is parallel to an axis direction of the reservoir 101 (i.e., the moving direction of the plunger 102); and the cylindrical structure 1031 is parallel to the plunger 102 movable along the axial direction of the reservoir 101 and is coaxially connected to the driving button 105 located below the injection button 103. In other words, pressing the injection button 103 can enable the plunger 102 and the driving button 105 to move side by side synchronously. The rotation sliding block 106 may be arranged coaxially below the driving button 105 by using the cylindrical structure 1031 of the injection button 103 as a shaft. When the injection button 103 is pressed, the rotation sliding block 106 can move downwards along the length direction of the cylindrical structure 1031 of the injection button 103 under the effect of the driving button 105. The spring 107 may be a retractable spring sleeved on a cylinder-shaped component 110 that is disposed on a pedestal 109 (e.g., a pedestal having a circular surface) and coaxial with the cylindrical structure 1031 of the injection button 103, and covered by a spring cap 108 at the top. The top surface of the spring cap 108 is in contact with the lower surface of the cylindrical structure 1031 of the injection button 103. When the injection button 103 is pressed, the spring 107 in the spring cap 108 is applied with a downward acting force, and the spring 107 is compressed to store mechanical energy. The base 104 may be a cylinder-like structure, and can wrap the driving button 105, the rotation sliding block 106, the spring 107, and the spring cap 108. A plurality of (e.g., more than 2) guide chutes 1041 are distributed uniformly on the circumference of the cylindrical structure. The guide chutes 1041 are in communication with each other at tail ends or lower ends (viewing from the direction facing FIG. 1), thus allowing the rotation sliding block 106 to slide and move from one guide chute into another adjacent guide chute. For example, the base 104 may be mounted on the pedestal 109, such that the linkage button 103, the driving button 105, the rotation sliding block 106, the spring cap 108, and spring 107 are together received in a space defined by the base 104 and the pedestal 109. In some other embodiments, the base 104 and the pedestal 109 may form an integrated structure.

The driving button 105 may be operatively connected to the injection button 103. When the injection button 103 is pressed to move downwards, the driving button 105 can also move downwards along with the injection button 103. The driving button 105 is provided with a plurality of protruding parts 1051 uniformly distributed along the circumference, and each protruding part 1051 is provided with a driving slope 1052. These protruding parts 1051 correspond to positions of the guide chutes 1041 of the base 104, and can be embedded into the corresponding guide chutes 1041.

The rotation sliding block 106 may be operatively coupled to the driving button 105, and is provided with a plurality of protruding parts 1061 uniformly distributed along the circumference. These protruding parts 1061 match with positions of the protruding parts 1051 of the driving button 105, each have a driven slope 1062 corresponding to the driving slope 1052, and can be embedded into the guide chutes 1041 of the base 104 correspondingly. As shown in FIG. 3, the protruding parts 1051 of the driving button 105 and the protruding parts 1061 of the rotation sliding block 106 may be embedded into the guide chutes 1041 of the base 104. Moreover, the driven slope 1062 of the rotation sliding block 106 can slide along the guide chute 1041 under the drive of the driving slope 1052 of the driving button 105.

The spring 107 may be operatively in contact with the rotation sliding block 106, and may be used as an energy storage component to store mechanical energy when the rotation sliding block 106 is applied with a pressing acting force to move downwards along the guide chute, and to release the stored mechanical energy when the rotation sliding block 106 moves to another guide chute, so as to apply a driving force (e.g., a restoring force of the spring) to the rotation sliding block 106 such that the rotation sliding block 106 returns to an unpressed state.

The protruding parts 1051 of the driving button 105 and the protruding parts 1061 of the rotation sliding block 106 are mounted in the guide chutes 1041 of the base 104 in a spline-like manner, and are kept in a free state as being supported by the spring 107, as shown in FIG. 4. When the user presses the injection button 103, the driven slope 1062 of the protruding part 1061 of the rotation sliding block 106 moves downwards along the guide chute under the effect of the driving slope 1052 of the driving button 105. As shown in FIG. 5, when moving to the tail end of the guide chute, the protruding part 1061 of the rotation sliding block 106 is separated from the guide chute and rotates to enter an adjacent guide chute. The spring 107 stores mechanical energy in the process when the rotation sliding block 106 moves downwards along the guide chute. When the rotation sliding block 106 slides to another guide chute, the spring 107 releases the stored mechanical energy to apply a driving force to the rotation sliding block 106, such that the rotation sliding block 106, together with the driving button 105, is restored to the free state shown in FIG. 4. The injection button 103 is pressed, the driving button 105 and the rotation sliding block 106 are driven to move downwards along the guide chute; and at the same time, the plunger 102 synchronously moving with the injection button 103 also generates the same displacement. The lengths of the guide chutes are the same, and therefore, the plunger 102 moves for a fixed distance each time, to push out a fixed amount of the fluid from the reservoir 101.

In the embodiment of the present invention, components such as the base 104, the driving button 105, the rotation sliding block 106, and the spring 107 arranged parallel to the reservoir match with each other to limit the plunger 102 to move for a fixed distance each time when the injection button 103 is pressed, thus greatly reducing the overall size of the apparatus, avoiding the complicated process for setting the infusion amount, and greatly facilitating the administration for users.

### [Second Embodiment]

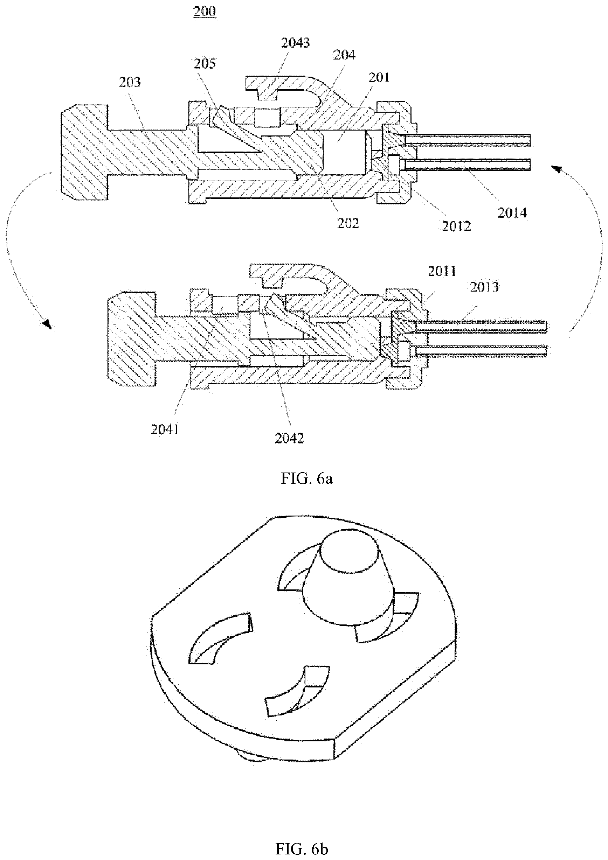

FIG. 6a is a schematic structural diagram of a fluid infusion apparatus used for administering a medicament to a patient according to a second embodiment of the present invention; and FIG. 6b is a schematic enlarged diagram of components according to the second embodiment of the present invention.

As shown in FIG. 6a, a fluid infusion apparatus 200 may include a reservoir 201, a plunger 202, an injection button 203, a matching part 204, and an elastic buckle 205. The plunger 202 may close one end of the cylindrical reservoir 201 and is movable in the reservoir 201 along an axial direction of the reservoir. The injection button 203 is connected to one end of the plunger 202 opposite to the end in contact with an inner cavity of the reservoir 201; and the elastic buckle 205 is arranged at a junction of the injection button 203 and the plunger 202. The matching part 204 is arranged parallel to a length extension direction of the reservoir 201, and corresponds to the position of the elastic buckle 205. The injection button 203 can drive the plunger 202 and the elastic buckle 205 to move synchronously. In some other embodiments, the plunger 202, the elastic buckle 205, and the injection button 203 may form an integrated structure.

The matching part 204 is arranged parallel to the reservoir 201. As shown in FIG. 6a, the matching part 204 may be arranged above the reservoir 201; for example, the matching part 204 may be sleeved on the outer surface of the reservoir 201. Alternatively, the matching part 204 and the reservoir 201 may form an integrated structure. The matching part 204 is provided with two openings 2041 and 2042 spaced at a fixed distance, and the elastic buckle 205 can be assembled in the openings 2041 and 2042.

When the injection button 203 is pressed, one end of the elastic buckle 205 pops up from the opening 2041 of the matching part 204, and moves for the fixed distance to reach the position of the second opening 2042 and be assembled to the second opening 2042. At the same time, the plunger 202 also correspondingly moves for the fixed distance, to force the fluid in the reservoir 201 to generate a pressure, thereby opening an output valve 2011 (e.g., a check valve) to push the fluid out from the reservoir 201 and deliver the pushed fluid to a patient through an output pipe 2013 (such as a hollow injection needle) in communication with the output valve 2011. As shown by the direction indicated via the arrow at the left of FIG. 6a, the reservoir 201 turns from a fluid filled state to a fluid drained state. The output valve 2011 may be a check valve that has the structure shown in FIG. 6b and is arranged at one end of the reservoir 201, opposite to the plunger 202. The check valve structure may be provided with two one-way channels; one may be used as an output valve, and the other may be used as an input valve. Such a structure facilitates the simplifying of the manufacturing process of the apparatus. The moving distance of the elastic buckle 205 from the opening 2041 to the opening 2042 is fixed; therefore, the distance of each move of the plunger 202 is also fixed, and the output fluid amount is also determined, thus avoiding manual setting of the infusion amount, simplifying the process for setting the infusion amount, and facilitating the administration for users.

The fluid infusion apparatus 200 may further include an energy storage component, such as a retractable spring, operatively connected to the elastic buckle 205. The energy storage component can store mechanical energy in the process when the elastic buckle 205 moves from the opening 2041 to the opening 2042. The energy storage component, the matching part 204, and the elastic buckle 205 together construct a displacement limiting mechanism. The displacement limiting mechanism cooperates with the injection button 203 to limit the plunger 202 to move for a predetermined distance within the reservoir 201.

The matching part 204 may further include a reset button 2043 matching with the opening 2042. Pressing the reset button 2043 can enable the reset button 2043 to be embedded into the opening 2042, to force the elastic buckle 205 to pop out from the opening 2042. The energy storage component releases the stored mechanical energy such that the elastic buckle 205 can be driven to move from the opening 2042 to the opening 2041. At the same time, the reservoir 201 may open an input valve 2012 (e.g., the check valve having the structure shown in FIG. 6b) to suck the fluid into the reservoir 201 through an input pipe 2014 (such as a hollow needle) in communication with the input valve 2012. As shown by the direction indicated via the arrow at the right of FIG. 6a, the reservoir 201 turns from a fluid drained state to a fluid filled state.

### [Third Embodiment]

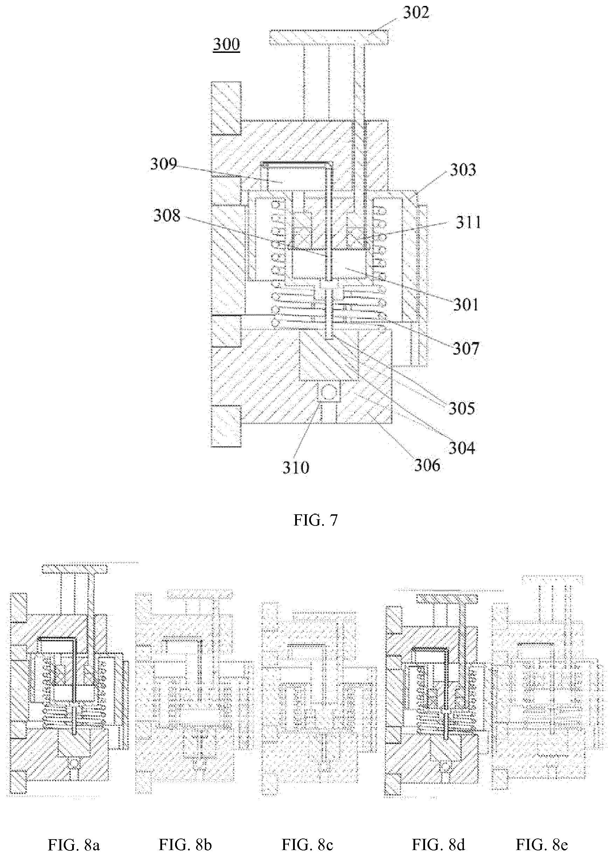

FIG. 7 is a schematic sectional diagram of a fluid infusion apparatus used for administering a medicament to a patient according to a third embodiment of the present invention; and FIG. 8a to FIG. 8e are schematic diagrams of operating states of the fluid infusion apparatus used for administering a medicament to a patient according to the third embodiment of the present invention.

As shown in FIG. 7, a fluid infusion apparatus 300 may include a reservoir 301, an injection button 302, an injector 303, a sealing element 304, and a first hollow piercing component 305. The reservoir 301 can store to-be-infused fluid (such as insulin). One end of the injection button 302 may be operatively connected to a plunger 311 (such as a silica gel piston) movable along an axial direction in the reservoir 301. For example, one end of the injection button 302 may be received in the plunger 311, and pressing the injection button 302 can push the plunger 311 to move in the reservoir 301. The reservoir 301 can be integrally received in the injector 303; for example, the reservoir 301 can be received in a cylindrical recessed part in the center of the injector 303. The injector 303 is movable, and can move synchronously with the reservoir 301. The sealing element 304 may be arranged outside of the injector 303, such as being arranged outside of one side of the injector 303 near an output channel 310. The first hollow piercing component 305 may be arranged between the reservoir 301 and the sealing element 304; a base end thereof can penetrate through the injector 303 to be in communication with the reservoir 301, and a front end thereof is sealed within the sealing element 304. When the injection button 302 is pressed, the first hollow piercing component 305 can move along with movements of the reservoir 301 and the injector 303.

The sealing element 304 may be a component having a sealing function, such as a silica gel plug, arranged at a fixed position outside of the injector 303, and does not move along with the movement of the injector 303. In the embodiment shown in FIG. 7, the sealing element 304 is arranged below the movable injector 303; for example, the sealing element 304 may be fixed to a supporting component 306 that supports the injector 303.

The first hollow piercing component 305 may be a steel needle having a hollow structure, a base end thereof is in communication with the reservoir 301 (for example, the base end can pierce through the injector 303 and is inserted into a chamber of the reservoir 301), and a front end thereof pierces through a part of the sealing element 304 outside the injector 303 and is sealed within the sealing element 304. In this sealed state, the fluid in the reservoir 301 cannot flow out through the first hollow piercing component 305. In the case that the front end of the first hollow piercing component 305 is sealed within the sealing element 304, when the injection button 302 is pressed, as the fluid in the reservoir 301 cannot be compressed, the injection button 302 drives the plunger 311 to move downwards (viewing from the direction facing FIG. 7) for a distance together with the reservoir 301, the injector 303, and the first hollow piercing component 305, till the first hollow piercing component 305 penetrates through the sealing element 304, such that the fluid in the reservoir 301 can be dispensed through the first hollow piercing component 305.

The fluid infusion apparatus 300 may further include an energy storage component 307, such as a retractable spring. The energy storage component 307 may be operatively connected to the injector 303, such as being arranged at the outer surface of a recessed part of the injector 303 for receiving the reservoir 301, and can move downwards along with the injector 303 and the reservoir 301 when the injection button 302 is pressed, so as to be compressed to store mechanical energy.

The fluid infusion apparatus 300 may further include a second hollow piercing component 308, such as a steel needle having a hollow structure. The second hollow component 308 may be bendable; for example, it can be bent by 90 degrees and be fixed at one side above the injector 303 by a fixing stopper 309 arranged above the injector 303 (viewing from the direction facing FIG. 7). The second hollow piercing component 308 may be in operative communication with the reservoir 301. For example, when the injection button 302 is pressed till the first hollow piercing component 305 penetrates through the sealing element 304, the second hollow piercing component 308 may be received in the plunger 311 and not in fluid communication with the reservoir 301, thereby allowing the fluid to flow out only through the first hollow piercing component 305. When the injection button 302 is released, the energy storage component 307 releases the mechanical energy to drive the injector 303 and the reservoir 301 as well as the first hollow piercing component 305 together to move upwards; and the first hollow piercing component 305 retracts into the sealing element 304, the second hollow piercing component 308 is in fluid communication with the reservoir 301, thereby allowing an external fluid to only flow into the reservoir 301 through the second hollow piercing component 308.

FIG. 8a to FIG. 8e show various operating states of the fluid infusion apparatus 300. As shown in FIG. 8a, the fluid infusion apparatus 300 is in a free state, the first hollow piercing component 304 is sealed within the sealing element 304; a front end of the second hollow piercing component 308 is located in the reservoir 301; the reservoir 301 is filled with fluid, and the fixing stopper 309 that fixes the second hollow piercing component 308 is in contact with the upper surface of the injector 303. Next, an external force is applied to press the injection button 302 downwards, as the fluid in the reservoir 301 cannot be compressed, the plunger 311 of the injection button 302 drives the reservoir 301, the injector 303 receiving the reservoir 301, and the first hollow piercing component 305 inserted into the reservoir 301 together to move downwards for a distance till the first hollow piercing component 305 penetrates through the sealing element 304. At the same time, the second hollow piercing component 308 retracts into the plunger 311, such that the reservoir 301 is in fluid communication with the external fluid output pipe 310; and the injector 303 will not move downwards continuously, as shown in FIG. 8b. The injection button 302 is pressed downwards continuously; the plunger 311 moves continuously downwards within the reservoir 301 to completely dispense the fluid in the reservoir 301 to the output pipe 310 through the first hollow piercing component 305. As shown in FIG. 8c, infusion of the fluid in the reservoir 301 is completed. In the process that the injection button 302 is pressed to enable the plunger 311 to move downwards, the energy storage component 307 stores mechanical energy. After the infusion of the fluid of the reservoir 301 is completed, the injection button 302 is released, and the energy storage component 307 will release the stored mechanical energy to drive the injector 303, the reservoir 301, and the first hollow piercing component 305 together to move upwards. As shown in FIG. 8d, the first hollow piercing component 305 moves upwards into the sealing element 304, so as to be sealed within the sealing element 304. The second hollow piercing component 308 retracting in the plunger 311 will be exposed from the plunger 311 as the plunger 311 moves upwards, such that the reservoir 301 is in fluid communication with the second hollow piercing component 308, allowing the external fluid to flow into the reservoir 301. At the same time, the upper surface of the movable injector 303 is in contact with the fixing stopper 309 that fixes the second hollow piercing component 308, to stop the injector 303 from moving upwards continuously. The mechanical energy released by the energy storage component 307 drives the plunger 311 to move upward continuously, allowing the fluid to flow into the reservoir 301 persistently through the second hollow piercing component 308, so as to fill the reservoir 301 with the fluid.

The fluid infusion apparatus 300 in the embodiment of the present invention can be manufactured as a wearable device, suchg as being adhered to the skin of a patient in a direction parallel to the length direction of the reservoir 301.

In the embodiment of the present invention, through the coordination among various components such as the injection button 302, the injector 303, the sealing element 304, the first hollow piercing component 305, the second hollow piercing component 308, and the energy storage component 307, a moving distance of the plunger is fixed in the injection and fluid filling process, thereby implementing dispensing of a fixed amount of the fluid by pressing the injection button once, avoiding the complicated process for setting the infusion amount, and facilitating the administration for users.

### [Fourth Embodiment]

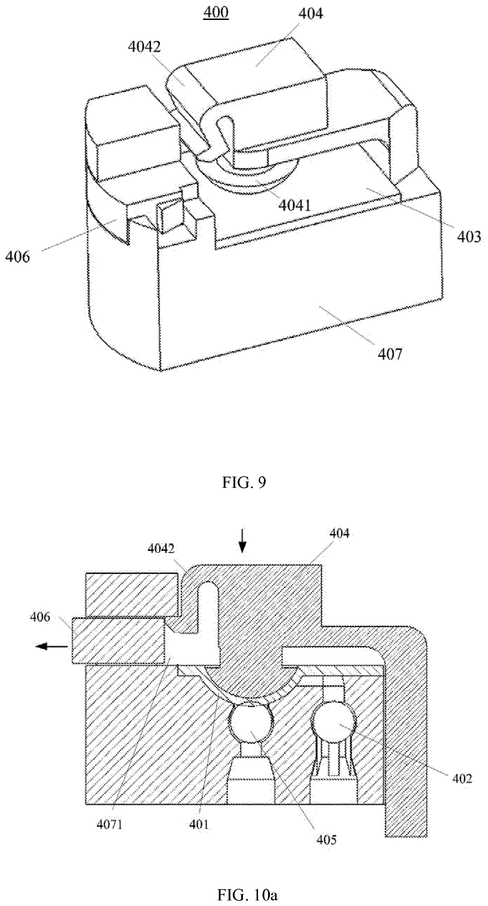

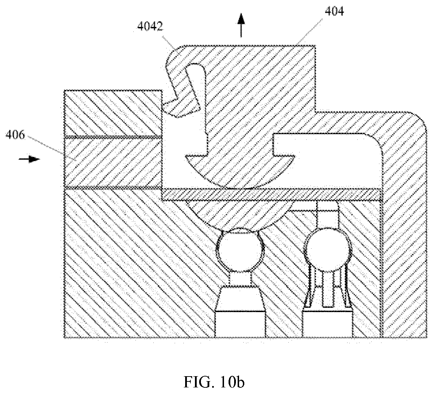

FIG. 9 is a schematic diagram of a fluid infusion apparatus used for administering a medicament to a patient according to a fourth embodiment of the present invention; and FIG. 10a and FIG. 10b are schematic diagrams of operating states of the fluid infusion apparatus used for administering a medicament to a patient according to the fourth embodiment of the present invention.

As shown in FIG. 9, FIG. 10a, and FIG. 10b, a fluid infusion apparatus 400 may include a reservoir 401, an output valve 402, a flexible film 403, an injection button 404, an input valve 405, a reset button 406, and a base 407. The reset button 406 and the injection button 404 may be mounted to the base 407, such as a shell-type base having a receiving space. Moreover, the reservoir 401, the output valve 402, and the input valve 405 may be received in the receiving space of the base 407, as shown in FIG. 9. An opening of the reservoir 401 may be aligned with the outer surface of the base 407; and the flexible film 403 covers the outer surface to isolate the reservoir 401 from the outside world. The reset button 406 may be mounted in a mounting hole 4071 at one side of the base 407, and is completely received in the mounting hole 4071 of the base 407 when the reset button is in a state not in contact with the injection button 404; and an end surface of the reset button being aligned with the outer surface of the base 407. The injection button 404 may be mounted at a position of the base 407 parallel to the reset button 406. When the injection button 404 is pressed, an embedding part 4042 of the injection button 404 can be embedded into the mounting hole 4071 of the base 407, so as to push a part of the reset button 406 out of the mounting hole 4071. The reservoir 401 may be used for storing infusion fluid, for example, it may be a bowl-shaped container shown in FIG. 10a and FIG. 10b, of which an outlet and an inlet are operatively connected to the output valve 402 (such as a ball valve) and the input valve 405 (such as a ball valve) respectively. For example, the output valve 402 may be arranged right below the reservoir 401; and the fluid of the reservoir 401 can flow to an injection syringe or the like through the output valve 402. The input valve 405 may be arranged at a position below the reservoir and parallel to the output valve 402. A fluid pipe may be arranged to connect the outlet of the input valve 405 with the bowl-shaped reservoir 401, such that an external fluid can flow to the reservoir 401 through the input valve 405. The reservoir 401 may be arranged below the injection button 404; specifically, the reservoir 401 may be arranged below a filling element 4041 of the injection button 404. The flexible film 403 is further arranged between the reservoir 401 and the filling element 4041. Specifically, as shown in FIG. 9, FIG. 10a, and FIG. 10b, the flexible film 403 covers the opening of the reservoir 401, such that one surface of the flexible film 403 is in contact with the reservoir 401, and the other surface can be in contact with the filling element 4041. The flexible film 403 may be a relatively thin film having resilience, such as a silica gel film, that can be used for enhancing the air tightness during filling, thereby isolating the fluid in the reservoir 401 from the outside world.

The injection button 404 may be arranged on the flexible film 403, and is provided with the filling element 4041 that fills the reservoir 401. In the embodiment shown in FIG. 9, the filling element 4041 is a bowl-like filler that can fill the bowl-shaped reservoir 401. In some other embodiments, the filling element 4041 may further be in other shapes matching the shape and the size of the reservoir 401, such as a cylindrical shape. Viewing from the direction facing FIG. 10a, a downward acting force is applied to the injection button 404; and the injection button 404 drives the filling element 4041 to move downwards to be pressed into the reservoir 401 together with the flexible film, such that the space of the reservoir 401 is filled by the filling element 4041, to force the fluid in the reservoir 401 to generate a pressure to open the output valve 402, thereby allowing the fluid of the reservoir 401 to be dispensed.

The injection button 404 may be operatively connected to the reset button 406. For example, the injection button 404 may be provided with an embedding part 4042 which can be embedded into the mounting hole 4071 of the reset button 406 on the base 407. When the injection button 404 moves downwards (for example, an acting force is applied along an arrow direction shown above the injection button 404 in FIG. 10a to enable the injection button 404 to move downwards) and fills the reservoir 401, the embedding part 4042 can be inserted into the mounting hole 4071 of the reset button 406 on the base 407, to push a part of the reset button 406 out of the outer surface of the base 407 along an arrow direction at the right of the reset button 406 in FIG. 10a.

As shown in FIG. 10b, an external force is applied along the arrow direction at the right of the reset button 406 to press the reset button 406, such that the part of the reset button 406 that is pushed out retracts into the surface of the base 407. At the same time, the embedding part 4042 of the injection button 404 is forced to pop out from the mounting hole 4071, and move upwards along an arrow direction above the injection button 404 in FIG. 10b, to drive the filling element 4041 together with the flexible film 403 to be lifted from the reservoir 401. A negative pressure generated in the reservoir 401 can force the output valve 402 in communication with the reservoir 401 to be closed, and force the input valve 405 to be opened to allow the fluid to flow into the reservoir 401, thus implementing fluid filling inside the reservoir 401.

In the embodiment of the present invention, through the coordination among various components such as the injection button 404, the flexible film 403, and the output valve 402, a fixed amount of the fluid in the reservoir 401 can be dispensed each time the injection button 404 is pressed, thus avoiding a complicated process of setting the infusion amount, simplifying the process for configuring the infusion amount, and greatly facilitating the administration for users.

It should be noted that the various aspects of the apparatus are described according to a specific order and a specific structural deployment; however, these are merely used for exemplification, and are not intended to limit the present invention. The subject claimed for protection is not limited to the described order and structural deployment. Those skilled in the art should understand that various modifications and equivalent replacements may be made for the invention without departing from the essence of the present invention. Therefore, the subject of the present invention claimed for protection is not limited to the specific embodiments disclosed above, and may also include all technical solutions falling within the protection scope of the claims and equivalent technical solutions. Moreover, unless otherwise specified, all terms in the claims should be understood based on their broadest and most reasonable meanings.

## Claims

1. A fluid infusion apparatus used for administering a medicament to a patient, comprising:
a reservoir, used for storing infusion fluid;
a plunger, located in the reservoir and limiting the infusion fluid together with the reservoir, wherein the plunger is configured to be movable along the reservoir;
an injection button, operatively and synchronously moving with the plunger and configured to be operable; and
a displacement limiting mechanism, arranged parallel to the reservoir, and configured to limit the plunger to move for a predetermined distance within the reservoir when the injection button is operated, thus dispensing a predetermined amount of the fluid stored in the reservoir.

2. The apparatus according to claim 1, wherein the displacement limiting mechanism comprises:
a base, provided with a plurality of guide chutes having equal lengths; and
a slider, operatively connected to the injection button, and configured to be pushed to slide along a first guide chute in the plurality of guide chutes when the injection button is pressed, and to be disengaged from the first guide chute and slides to an adjacent second guide chute in the plurality of guide chutes when the injection button is released.

3. The apparatus according to claim 2, wherein the displacement limiting mechanism further comprises:
an energy storage component, operatively connected to the slider, and configured to store mechanical energy when the slider slides along the first guide chute and to release the mechanical energy such that the slider returns to an unpushed state when the slider slides to the second guide chute.

4. The apparatus according to claim 3, wherein the slider comprises:
a driving button, operatively connected to the injection button and provided with a plurality of first protruding parts uniformly distributed along a circumference and embeddable into the guide chutes, wherein the first protruding part is provided with a driving slope; and
a rotation sliding block, operatively coupled to the driving button and the energy storage component and provided with a plurality of second protruding parts uniformly distributed along a circumference and embeddable into the guide chutes, wherein the second protruding part is provided with a driven slope corresponding to the driving slope.

5. The apparatus according to claim 1, wherein the displacement limiting mechanism comprises:
a matching part, provided with a first opening and a second opening spaced at a fixed distance; and
an elastic buckle, synchronously moving with the injection button and the plunger, and configured to pop out from the first opening and move for the fixed distance to be assembled to the second opening when the injection button is pressed.

6. The apparatus according to claim 5, wherein the matching part further comprises:
a reset button, matching with the second opening and configured to be pressed such that the elastic buckle pops out from the second opening.

7. The apparatus according to claim 6, wherein the displacement limiting mechanism further comprises:
an energy storage component, operatively connected to the elastic buckle, and configured to store mechanical energy when the elastic buckle moves from the first opening to the second opening, and to release the mechanical energy when the elastic buckle pops up from the second opening such that the elastic buckle moves for the fixed distance to be assembled to the first opening.

8. A fluid infusion apparatus used for administering a medicament to a patient, comprising:
a reservoir, used for storing a to-be-infused fluid;
an injection button, operatively connected to a plunger moving along the reservoir, and configured to be operable;
an injector, used for receiving the reservoir and being synchronously movable with the reservoir;
a sealing element, arranged outside of the injector; and
a first hollow piercing component, a base end of the first hollow piercing component being in communication with the reservoir, a front end of the first hollow piercing component being sealed within the sealing element, and the first hollow piercing component being synchronously movable with the reservoir, the injection button, and the injector,
wherein when the injection button is pressed, the reservoir, the injector, and the first hollow piercing component are driven to move synchronously such that the first hollow piercing component penetrates through the sealing element to dispense the fluid in the reservoir.

9. The apparatus according to claim 8, further comprising:
an energy storage component, operatively connected to the injector, and configured to store mechanical energy when the injection button is pressed and to release the mechanical energy when the injection button is released such that the first hollow piercing component is sealed within the sealing element.

10. The apparatus according to claim 9, further comprising:
a second hollow piercing component, in operative communication with the reservoir, and being capable of inputting a fluid to the reservoir when it is in communication with the reservoir.

11. The apparatus according to claim 10, wherein the second hollow piercing component is further capable of retracting within the plunger when the injection button is pressed.

12. A fluid infusion apparatus used for administering a medication to a patient, comprising:
a reservoir, used for storing infusion fluid;
an output valve, operatively connected to an outlet of the reservoir;
a flexible film, covering the reservoir; and
an injection button, located on the flexible film and provided with a filling element filling the reservoir, and configured to be pressed such that the filling element, together with the flexible film, fills the reservoir so that the fluid pressure within the reservoir forces open the output valve, enabling the fluid in the reservoir to be dispensed.

13. The apparatus according to claim 12, further comprising:
an input valve, operatively connected to an inlet of the reservoir; and
a reset button, operatively connected to the injection button and configured to be pressed such that the filling element, together with the flexible film, is lifted from the reservoir, forcing the output valve to be closed and the input valve to be opened, thus inputting a fluid to the reservoir.

14. The apparatus according to claim 12 or 13, wherein the output valve comprises a ball valve.

15. The apparatus according to claim 12 or 13, wherein the input valve comprises a ball valve.
